# EUROPEAN PATENT SPECIFICATION

(11) **EP 1 223 914 B1**
(45) Date of publication and mention of the grant of the patent: **01.09.2004**
(21) Application number: 00971346.2
(22) Date of filing: 09.10.2000
(51) Int. Cl.: A61K 9/00, A61K 31/55

(54) **ORALLY DISTINTEGRATING COMPOSITION COMPRISING MIRTAZAPINE**
IM MUND ZERFALLENDE ZUSAMMENSETZUNG MIT MIRTAZAPIN
NOUVELLE FORMULATION DE MIRTAZAPINE

(30) Priority: 13.10.1999 EP 99203338
(43) Date of publication of application: 24.07.2002
(73) Proprietor: Akzo Nobel N.V., 6824 BM Arnhem (NL)
(72) Inventor: DE NIJS, Henrik, NL-5343 XC Oss (NL)
(74) Representative: Kraak, Hajo
(86) International application number: PCT/EP2000/009863
(87) International publication number: WO 2001/026621

(56) References cited:
- EP-A- 0 313 328
- EP-A- 0 436 252
- EP-A- 0 737 473
- US-A- 5 178 878
- US-A- 5 464 632
- FAWCETT J ET AL: "Review of the results from clinical studies on the efficacy, safety and tolerability of mirtazapine for the treatment of patients with major depression." JOURNAL OF AFFECTIVE DISORDERS, (1998 DEC) 51 (3) 267-85, XP002127404
- PUZANTIAN T. ET AL: "Mirtazapine, an antidepressant." AMERICAN JOURNAL OF HEALTH-SYSTEM PHARMACY, (1998) 55/1 (44-49)., XP002127405
- VOORTMAN, G. ET AL: "Bioavailability of mirtazapine from Remeron tablets after single and multiple oral dosing" HUM. PSYCHOPHARMACOL. (1995), 10(SUPPL. 2), S83-S96, XP002112718

## Description

The invention relates to a dosage unit for peroral administration, comprising a pharmaceutical formulation of mirtazapine as an active substance and pharmaceutically acceptable excipients.

Mirtazapine (Remeron®) is known as a medicine for the treatment of, *inter* alia, depression. As an anti-depressant, mirtazapine is in a class of its own, recognized as a noradrenergic and specific serotonergic antidepressant (NaSSA) that enhances both noradrenergic and serotonergic neurotransmission by means of antagonism at presynaptic α₂ adrenoceptors while blocking postsynaptic serotonin 5-HT₂ and 5-HT₃ receptors.

Dosage units for peroral administration of mirtazapine are known in the form of conventional tablets. The strength of the medicinally active ingredient ranges from 15 to 45 mg mirtazapine. These known tablets are generally acceptable as far as their medicinal activity is concerned, and mirtazapine has been proven to be a safe, well-tolerated and effective anti-depressant. Nevertheless, as would seem unavoidable in the area of anti-depressant drugs, also mirtazapine shows room for improvement. Such an improvement is sought, int.al., in the diminishing of side-effects. For mirtazapine, these side-effects particularly include appetite increase, weight gain, daily somnolence, sexual dysfunction, gastro-intestinal side-effects, edema, restless legs, dry mouth, excessive sedation. Specifically for mirtazapine it were also desired to further improve some of its beneficial effects, e.g. better sleep, and a stronger anxiolytic effect, and to achieve an earlier onset of antidepressant action. The latter is of particular importance in psychiatry. The known antidepressants generally require a period of several weeks before anti-depressant activity is displayed. It is desired to reduce this period, preferably to less than one week.

Improved dosage forms of mirtazapine have been disclosed in the art. EP 436 252 which discloses powdered particulates or granules useful for the preparation of an oral liquid pharmaceutical formulation of mirtazapine. These granules, when introduced into an appropriate quantity of water and agitated, provide readily drinkable suspensions of mirtazapine. In EP 431 663 a stabilized solution of mirtazapine is disclosed which is suitable for parenteral or peroral administration. In copending, non-prepublished patent application PCT/EP 99/02277, another aqueous peroral solution or suspension of mirtazapine is disclosed. This product is characterised by having a concentration of mirtazapine in the range of from 5 to 110 mg/ml, a pH in the range of from 2.0 to 3.5, and comprising a thickening agent. Disclosed is also a pre-mix from which this oral solution or suspension can be produced by adding water, or by mere contact with the saliva of the person to be treated. It is noted that the pre-mix must produce the solution or suspension as described in PCT/EP 99/02277, i.e. the pre-mix must be of such a nature as to render a solution or suspension having the above pH requirements. In view of the buffer capacity of human saliva, this means that if a pre-mix is intended to bring about a suspension having a pH as low as 2.0-3.5, it must contain a relatively large amount of acid.

Even if it is recognized that such alternative formulations of mirtazapine may be useful, the present invention explicitly is concerned with providing discrete dosage units for peroral administration which can fully serve as a substitute for the known tablets marketed under the tradename Remeron®. Thus it is an object of the present invention to bring about an improvement of mirtazapine when administered in discrete dosage units for peroral administration which have the same strength as, and are bio-equivalent with, the conventional tablets.

To this end, the invention resides in a dosage unit for the peroral administration of mirtazapine as defined in the opening paragraph, the dosage unit being of the orally-disintegrating type, and wherein the formulation comprises means which substantially prevent mirtazapine from being released orally (i.e. in the mouth rather than in the gastrointestinal tract).

The discrete oral dosage units thus provided have the same strengths as, and - unobviously so when viewed without the benefit of hindsight - are bioequivalent with, the known tablets of 15, 30 and 45 mg. Surprisingly, and without intending to give any impression as if all side-effects can be diminished in all patients, the dosage units of the invention can be used to bring about improved effects of mirtazapine, such as onset of anti-depressant action within one week, and diminished side-effects such as weight gain and excessive sedation.

The invention is disclosed in more detail hereinafter with reference to the various characteristics of the dosage units as defined above.

Dosage units are physically discrete units suitable as unitary dosages for a human patient, each containing a predetermined quantity of active material, in this case 15-45 mg of mirtazapine, for instance tablets, pills, capsules and the like.

Peroral administration means that the dosage unit is administered via the mouth of the patient, in whichever manner thereafter the drug is released and absorbed.

A pharmaceutical formulation is a composition comprising an active substance, either on its own (in which case the formulation equals the active substance), or in association with other components which are not recognized as medicinally active agents, but merely contribute to containing the active substance. Such (inert) ingredients typically are diluents, fillers, stabilisers, and other adjuvants.

Mirtazapine, which is the compound 1,2,3,4,10,14 β-hexahydro-2-methylpyrazo[2,1-a]pyrido [2,3-c][2] benzazepine, is intended to include the compound *per* se as well as pharmaceutically acceptable salts thereof. The compound can be prepared as disclosed in United States Patent No. 4,062,848 to van der Burg.

It will be appreciated that mirtazapine contains a centre of chirality. The present invention includes each of the individual (R) and (S) enantiomers of mirtazapine and it salts in a form substantially free from the other enantiomer (i.e. having an enantiomeric purity of greater than 95% and preferably greater than 99%), as well as mixtures of the enantiomers in any proportion including a racemic mixture.

The term "pharmaceutically acceptable excipients" is widely known and used in the art as referring to all those, generally inert materials which a pharmaceutical formulation may contain in addition to the active substance, and which are recognized to be safe for this purpose. Such excipients include carriers, diluents, binders, lubricants, glidants, disintegrants, coloring agents, flavoring agents, stabilizers, and the like. These types of ingredients are known to the skilled person, and do not require further elucidation. Various textbooks on this matter exist, see, e.g., the standard English language text Gennaro et al., Remington's Pharmaceutical Sciences, (18th ed., Mack Publishing Company, 1990, especially Part 8: Pharmaceutical Preparations and Their Manufacture), in which methods of making tablets, capsules and pills and their respective components are described. Peroral solid dosage forms, with which the present invention is mainly concerned, are described in chapter 89.

An "orally disintegrating dosage unit" is a term which distinguishes the type of dosage units with which the invention is concerned from conventional tablets. The term thus refers to dosage units which, upon peroral administration, disintegrate in the mouth and/or the esophagus, rather than in the stomach or the gastro-intestinal tract. This can generally be associated with a disintegration time of less than 60 seconds. Examples include effervescent tablets, fast-dissolving, fast-melting tablets, sublingual tablets.

For the purpose of more clearly defining the formulation according to the invention, reference is made to the following three fundamentally different categories of solid dosage forms that can be distinguished, all having in common that they serve to administer a drug through the mouth:
(a) dosage units that are to be swallowed and disintegrate in the stomach or gastro-intestinal tract so as to release the drug there; absorption of the drug takes place through the gastro-intestinal route; examples include classic compressed tablets and granulate-filled capsules;
(b) orally-disintegrating sublingual or buccal dosage units; these cannot be effectively swallowed or chewed, as they disintegrate rapidly in the mouth upon contact with saliva; they release the drug locally in the sublingual or buccal pouch; absorption of the drug takes place through the sublingual or buccal mucosa; examples include sublingual and buccal tablets;
(c) orally disintegrating dosage units which cannot be swallowed integrally, but disintegrate in the mouth spontaneously or through chewing or sucking; from the disintegrated particles the drug is released in the esophagus or the stomach, whereupon absorption occurs via the gastro-intestinal tract; examples: effervescent tablets or multiparticulate tablets, each with a coated or otherwise encapsulated drug, or with a drug which inherently is not absorbed via the mucosa.

According to the invention, the orally-disintegrating tablets are those of category (c), i.e. of such a nature that mirtazapine is absorbed, at least for the greater part, in the same way as through the normal peroral route. In the case of the sublingual or buccal routes, which are designed to have direct, mucosal absorption of an active substance rather than via the hepato-gastro-intestinal first-pass metabolism, the tablet provided will not normally be bio-equivalent with the regular peroral tablets. According to the invention a mirtazapine dosage form is provided which is fundamentally comparable with the existing regular compressed tablets, however with the unexpected benefits found with oral disintegration.

Orally-disintegrating pharmaceutical formulations in themselves are known to the person skilled in the art. Reference is made, e.g., to the disclosures in EP 636 364, EP 737 473, US 5,178,878, US 6,024,981 and US, 5,464,632. All of these disclosures refer to a formulation of particles of the active ingredient, wherein the particles are held together in such a way as to rapidly disintegrate upon oral administration. In EP '364 the disintegration is brought about by a water-disintegratable, compressible carbohydrate and a binder. In US 5,178,878 the disclosed pharmaceutical dosage form incorporates microparticles provided in a tablet with an effervescent disintegration agent. When the tablet is taken orally, the effervescent disintegration agent aids in the rapid disintegration of the tablet and permits release of the microparticles, and swallowing of the microparticles, before the pharmaceutical ingredient is released from the microparticles. In US 5,464,632 another rapidly disintegratable multiparticulate tablet is disclosed. It contains an excipient mixture which is suitable for imparting a disintegration rate such that the table disintegrates in the mouth in less than 60 seconds, wherein the active substance is present in the form of coated microcrystals or microgranules. Another pharmaceutical formulation which may be suitable as an orally disintegrating formulation in the present invention is disclosed in US 5,225,197. This is a chewable tablet comprising a medicament dispersed in a chewable base, such as mannitol, together with an effervescent couple, such as citric acid-sodium bicarbonate.

The means which substantially prevent mirtazapine from being released orally have a function in preventing mirtazapine from being absorbed as in the case of a sublingual or buccal formulation. Although, in theory, e.g. a salt of mirtazapine might be devised which could prevent oral absorption, according to the present invention the compound itself remains unaltered, and the means to prevent oral release actually shields mirtazapine until it has been swallowed. Such means are known, not only from disclosures on orally disintegrating dosage units, but also from disclosures on sustained release compositions, such as EP 313 328. The suitable means is a coating layer on the active agent, which is of such a type that it will remain intact in the mouth, and will dissolve, disintegrate (or which will otherwise open up), so as to free the active substance when desired. Although truly sustained-release coatings are not preferred in view of the desired bioequivalence of the dosage units according to the present invention with the conventional tablets, the person skilled in the art will be able, without undue experimentation, to try such coatings on mirtazapine and to establish the required release time in the stomach by means of a simple dissolution test in an acidic environment. Such tests are described in the aforementioned Gennaro reference, chapter 31.

Suitable coatings for the active substance are described, among others, in the aforementioned disclosures on orally disintegrating dosage units, EP 636 364, US 5,178,878, and US 5,464,632. Thus EP '364 refers to several taste-masking compositions which are suitable as coatings preventing the oral release of mirtazapine as well. Reference is made to the methods and materials disclosed in EP 636 364 rather than repeating this disclosure here in full. These coatings may be blends, as amply described therein. It is preferred to compose a coating of one material only, which according to EP'364 can be hydroxy propyl cellulose, hydroxy ethyl cellulose, Eudragit® E100 (methylaminoethyl-methacrylate and neutral methacrylic acid esters available from Rohm Pharma GmbH), or hydroxy propyl methyl cellulose. In US 5,178,878 similar protective materials are disclosed, including other Eudragit® materials.

It should be noted that the dosage units of the invention do not need to have the undesirably high amount of acid which would be needed if, upon contact with saliva, a pH of 2 to 3.5 would have to be reached. Essentially, in the oral solution or suspension of PCT/EP 99/02277, the low pH is required to prevent a local anaesthetic effect. According to the present invention, the means which prevent mirtazapine from being released orally, at the same time can function as a taste-mask as well as a mask for the local anaesthetic effect.

In view of the alkaline nature of mirtazapine, the compound has been found to be surprisingly suitable in being provided with a polymeric coating which has a closed structure in the neutral, slightly alkaline pH of 7.4 found in the mouth, and which has a more open structure in the highly acidic environment of the stomach, thereby releasing mirtazapine in essentially the same way as the conventional tablets. Such polymers, which are believed to exert their effect by undergoing a conformational change depending on pH, are known to the person skilled in the art. The aforementioned Eudragit® E100 is preferred.

As noted above, orally disintegrating dosage units frequently comprise small particles, microparticles, or other particulate material. Said particles may include the active ingredient. In that case, the particle itself can function to prevent the oral release of the mirtazapine contained therein. Alternatively, which is preferred in the case of mirtazapine, the active substance is coated onto such small particles. It is further preferred that the small particles are so-called "non-pareils," which are known in the art as highly uniform sugar particles having a neat spherical (ball-like) shape. It is known in the art to use such non-pareils for coating drugs thereon. As indicated above, the mirtazapine on its turn is preferably coated with a polymer, such as Eudragit E100.

In summary, the oral dosage unit of the invention is the new combination of the anti-depressant mirtazapine, in a dosage unit of the orally-disintegrating type. The dosage unit, while rapidly disintegrating upon oral administration, is bioequivalent with conventional tablets, but can lead to advantages in terms of side-effects and onset of action. It should be noted that onset of antidepressant action is not normally linked to disintegration-time, since the former normally is regarded in terms of weeks, while a tablet is given daily. Antidepressant action can be determined in several ways, chiefly through the Hamilton Depression Rating Scale, the Clinical Global Impression Scale, and/or the Montgomery Asberg Depression Rating Scale.

The invention also resides in the use of mirtazapine for the manufacture of a medicament in the treatment of depression, wherein the medicament is a dosage unit of the aforementioned orally disintegrating type, and wherein mirtazapine is substantially prevented from being released orally. The invention further is a method of treatment involving the administration, to a human patient in need of such treatment, of at least one dosage unit of the aforementioned orally disintegrating type, and wherein mirtazapine is substantially prevented from being released orally. In all cases, it is preferred if mirtazapine is not released orally in more than 90%, and most preferably is not released at all. The dosage unit referred to thus preferably comprises mirtazapine provided with a coating, as described hereinbefore.

The invention is further explained with reference to the following examples.

### Example 1

Orally disintegrating tablets each containing 30 mg of mirtazapine are prepared using the following excipients: mannitol, crospovidone, sodium bicarbonate, citric acid, microcrystalline cellulose, aspartame, magnesium stearate, flavour, non-pareils, povidone, hydroxypropyl methyl cellulose, Eudragit E100. The non-pareils are provided with a coating layer of mirtazapine in conventional manner. These mirtazapine-coated particles are then coated with a layer of Eudragit E100 in a manner as described in EP 636 364. The resulting coated particles are subsequently incorporated with the remaining excipients into an effervescent formulation in accordance with Example I of US 5,178,878.

### Example 2

Test of the bioequivalence of the mirtazapine orally disintegrating tablet according to Example 1, in comparison with the marketed tablet containing the same amount of mirtazapine (30 mg), which is sold under the tradename Remeron®, after a single oral administration under fasting conditions.

Methodology: open, randomized, two-treatment, two-period, two-sequences crossover trial.
Number of subjects: forty subjects were included (20 males and 20 females), all of which completed the trial. Forty subjects received all two treatments.
Treatment T (test): single oral dose of one tablet according to Example I under fasting conditions. No water was given with the tablet.
Treatment R (reference): single oral dose of one tablet of Remeron® (containing 30 mg of mirtazapine) under fasting conditions, followed by 200 mL of non-carbonated water.
Duration of the treatment: two single dose administrations, separated by a washout period of at least two weeks.
Pharmacokinetics: blood samples for mirtazapine concentrations determinations were taken pre-dose, 15, 30, 45, 60, and 90 minutes and 2, 2.5, 3, 4, 6, 8, 12, 16, 24, 36, 48, 72, and 96 hours after mirtazapine administration. From the individual plasma concentration-versus-time curves following parameters were derived: Cₘₐₓ, tₘₐₓ, AUC₀₋ₜₗₐₛₜ, t_{1/2}, λ_{z} and AUC_{0-∞}. From the primary parameters Cₘₐₓ, AUC₀₋ₜₗₐₛₜ and AUC_{0-∞} parametric point estimates of the true ratio "test/reference" with their 90% confidence intervals (multiplicative model) derived from the Analysis of Variance were calculated. Parameters were considered bio-equivalent if the 90% confidence interval is fully within the acceptance range (0.80-1.25). For tₘₐₓ classical hypothesis testing was performed using an ANOVA on ranks.
Results: see Table 1.
Conclusion: The Reference formulation and the Test formulation are bioequivalent with respect to the maximum concentration, the area under the curve from zero up to the time of the last measurable concentration and the area under the curve from time zero up to infinity.

### Example 3

Test of the onset of antidepressant activity, as well as body weight, sexual functioning, and other effects associated with mirtazapine, as a result of administering a dosage unit according to the invention to human subjects with a Major Depressive Episode (according to DSM-IV criteria). Subjects are assessed to find antidepressant activity within the first and second week of treatment. Further assessments are done biweekly.

**Table 1**

| **Results of Bioequivalence Testing** | | | |
|---|---|---|---|
| Parameter | Point estimate | 90 % C.I | Conclusion |
| Cₘₐₓ | 1.072 | 0.948 - 1.212 | Bioequivalent |
| AUC_{0-dast} | 1.103 | 1.052-1.156 | Bioequivalent |
| AUC_{0-∞} | 1.101 | 1.049 - 1.156 | Bioequivalent |

## Claims

1. A dosage unit for peroral administration, comprising a pharmaceutical formulation of mirtazapine or a pharmaceutically acceptable salt thereof as an active substance, pharmaceutically acceptable excipients, and a layer, which is coating mirtazapine and which substantially prevents mirtazapine from being released orally, wherein the dosage unit is of the orally-disintegrating type and the layer disintegrates in an acidic environment.

2. A dosage unit according to claim 1, **characterized in that** the layer is a polymer.

3. A dosage unit according to claim 2, **characterized in that** the polymer is made from methylaminoethyl-methacrylate and neutral methacrylic ester monomers.

4. A dosage unit according to claim 3, **characterized in that** the mirtazapine on its turn is coated onto inert particles.

5. A dosage unit according to claim 4, **characterized in that** the particles are non-pareils.

## Patentansprüche

1. Eine Dosiseinheit zur peroralen Verabreichung umfassend eine pharmazeutische Formulierung von Mirtazapin oder eines pharmazeutisch verträgliches Salzes davon als Wirkstoff, pharmazeutisch verträgliche Arzneistoffträger und eine Schicht, die Mirtazipin beschichtet und die im wesentlichen verhindert, dass Mirtazapin oral freigesetzt wird, wobei die Dosiseinheit von dem oral sich zersetzenden Typ ist und sich die Schicht in einer sauren Umgebung zersetzt.

2. Eine Dosiseinheit gemäss Anspruch 1, **dadurch gekennzeichnet, dass** die Schicht ein Polymer ist.

3. Eine Dosiseinheit gemäss Anspruch 2, **dadurch gekennzeichnet, dass** das Polymer aus Methylaminoäthyl-Methacrylat und neutralen Methacrylester-Monomeren gemacht ist.

4. Eine Dosiseinheit gemäss Anspruch 3, **dadurch gekennzeichnet, dass** das Mirtazapin seinerseits auf inerte Partikel beschichtet ist.

5. Eine Dosiseinheit gemäss Anspruch 4, **dadurch gekennzeichnet, dass** die Partikel Nonpareils sind.

## Revendications

1. Une forme galénique unitaire pour l'administration perorale, comprenant une formulation pharmaceutique de mirtazapine ou d'un sel de celle-ci acceptable du point de vue pharmaceutique en tant que composant actif, des excipients acceptables du point de vue pharmaceutique, et une couche qui recouvre la mirtazapine et empêche sensiblement la libération de la mirtazapine orale, où la forme galénique est du type à désagrégation orale et la couche se désagrège dans un environnement acide.

2. Une forme galénique selon la revendication 1, **caractérisée en ce que** la couche est un polymère.

3. Une forme galénique selon la revendication 2, **caractérisée en ce que** le polymère est préparé à partir de monomères de méthacrylate de méthylaminoéthyle et d'ester méthacrylique neutre.

4. Unité de dosage selon la revendication 3, **caractérisée en ce que** la mirtazapine est elle-même appliquée sur des particules inertes.

5. Unité de dosage selon la revendication 4, **caractérisée en ce que** les particules sont des non-pareils.
